# EUROPEAN PATENT APPLICATION

(11) **EP 0 565 315 A2**
(43) Date of publication of application: **13.10.1993**
(21) Application number: 93302614.8
(22) Date of filing: 02.04.1993
(51) Int. Cl.: G03C 7/413, C07C 213/00

(54) **Colour development composition and method of processing silver halide colour photographic material using the same**

(30) Priority: 08.04.1992 JP 115501/92; 16.04.1992 JP 122591/92
(71) Applicant: CHUGAI PHOTO CHEMICAL CO. LTD., Chiyoda-ku, Tokyo (JP)
(72) Inventor: Sigemori, Nobuki, In Chugai Photo Chemical Co.Ltd., Matsudo City, Chiba Prefecture (JP)
(74) Representative: Lyons, Andrew John

(57) **Abstract**

A color developer composition contains a reaction product obtained by causing a reaction of an amine compound of 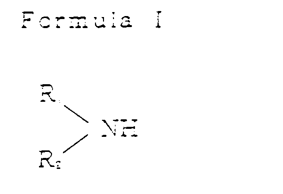
where R₁ and R₂ independently represent a member of the group consisting of a hydrogen atom, an alkyl group, an alkenyl group and an allyl group, these groups being capable of being substituted by a member of the group consisting of a hydroxyl group, an alkoxyl group and a carboxyl group, R₁ and R₂ being capable of jointly forming a hetero-ring, R₁ and R₂ being capable of forming a united bis compound, R₁ and R₂ being not both hydrogen atoms or 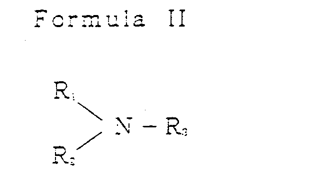
where R₁ to R₃ independently represent a member of the group consisting of an alkyl group, an alkenyl group and an alkyl group, these groups being capable of being substituted by a member of the group consisting of a hydroxyl group, an alkoxyl group and a carboxyl group, R₁ and R₂ being capable of jointly forming a hetero-ring, R₁ and R₂ being capable of forming a united bis compound and hydrogen peroxide (hydrogen peroxide water) and without separation of the reaction product or by reaction of an amine compound of the Formula II and hydrogen peroxide (hydrogen peroxide water) then causing a reaction of the reacted system and an amine of the Formula I and without separation of the reaction product.

## Description

### Field of the Invention

This invention relates to color developer compositions used for processing silver halide color photographic material and also to a method of processing silver halide color photographic material using the same. More specifically, the invention relates to color developer compositions, which are excellently stable in use as color developer solution and provide for satisfactory environment of use, and also a method of processing silver halide color photographic material using the same.

### Prior Art

Among the basic process of color photographic material that are effected after exposure of the photographic material to a light image, are those of color developing, silver bleaching, silver halide fixing, bleaching fixing, in which a bleaching process and a fixing process are effected at a time, water washing, rinsing which is effected in lieu of the water washing process, stabilizing and drying. In addition, there are additional processes, which are executed to permit each process solution to be used stably or to increase the physical strength and stability of the processed photographic material. Up to date, the process of silver halide color photographic material is used extensively to obtain color prints from color negative films. There are also processes for obtaining positive color films and prints such that final images are obtained directly. These process basically comprise a process for obtaining a color negative film or print and a monochromatic developing process. Further, users of color prints have a demand of observing print as soon as possible and desire the production of prints in as short period of time as possible. To meet this demand, it is necessary to permit quick treatment.

In recent years, owing to improvements it is possible to quickly treat the silver halide photographic materials owing to improvements therein. In addition, easy process of a large amount of the material is possible with automatic developing apparatus. Further, the automatic developing apparatus has been improved, and a variety of small-size and inexpensive apparatuses are used.

The color developer solution used for the process of the silver halide color photographic material, uses a principal color developer material of aromatic primary amine type such as paraphenylene diamine. In addition, sulfites and hydroxylamine are used as anti-oxidizing agent for preventing the oxidization of the principal color development material by air and so forth, and alkali metal salts such as carbonates and phosphates and hydroxides of alkali are used as alkali-maintaining agent for maintaining the color developer material to be alkaline. Further, a water softening agent and other additives are used, if necessary.

Recently, silver halide color photographic material for color paper uses silver chloride bromide emulsion having high silver chloride to permit quick process. Hydroxylamine has been used as the chloride bromide photographic material with high silver chloride content. In this case, however, the color density is reduced. In case when the sole sulfite is used, which is again an anti-oxidization agent, the stability of the color developer solution can be maintained to a certain extent. This color developer solution, however, results in fogging after lapse of a process time and therefore can not be used. Accordingly, N,N-diethylhydroxylamine is generally used in lieu of hydroxylamine. N,N-diethylhydroxylamine has a character of reducing the color density like but not as much as that of hydroxylamine. As a demerit, however, N,N-diethylhydroxylamine has a peculiar odor. This odor is undesired for the process operation and in the environmental standpoint such as when the process is done in a part of a general shop called development shop. Therefore, it is undesired to use this color developer material. Hydroxylamine used as the anti-oxidization agent for color negative film color developer solution, on the other hard, is a deadly compound, that is, it is a compound not desired to be used.

For carrying out the above photographic process, automatic developing apparatus is used in order to quickly treat a large quantity of photograph. In actual practice, the process of a given quantity of photographic material is carried out while replenishing with a necessary quantity of process solution as replenishment solution. The replenishment solution is prepared in a predetermined quantity. This means that a certain period of time is elapsed until it the solution is used up. Therefore, the status of the solution varies in the initial stage of use and after lapse of a certain period of time. In such a case, changes are provided in the quality of the processed photograph. For this reason, it is necessary to ensure satisfactory stability of the replenishment solution. Further, the process solution used in the automatic developing apparatus is discharged in a quantity corresponding to the quantity of the replenishment solution, with which the process solution is replenished. This means that a certain period of time is also elapsed until the completion of the replenishment, and the stability during is period is again necessary.

A photographic process dealer, who accepts a request of photographic process from a photographer, has to effect the desired process in a short period of time. To effect the color development quickly, a process solution having a high temperature character is used as the color developer solution. In such a case, the process solution is naturally subject to great deterioration. That is, if the solution is used for long time, its composition is changed to cause reduction of the development quality, change in the image quality and overall paper contamination. Therefore, a satisfactory result can not be obtained. This undesired result is presumably attributable to the deterioration of the principal color development material and anti-oxidization agent and also to the accumulation of substances issuing from the photographic material being processed. Further, when the automatic developing apparatus is used for the developing process, waste solution is produced in correspondence to the amount of photographic material that is processed. In view of the resources saving and adverse environmental effects of the waste, it is desired to reduce the amount of the waste, i.e., the amount of the replenishment solution. With reduction of the amount of replenishment, the time until the prepared replenishment solution is used up is extended, and also the time, during which the solution is retained in the automatic developing apparatus, is extended. The deterioration of the process solution proceeds in accordance with this time interval.

The inventors conducted researches and investigations concerning a color developer composition for processing silver halide color photographic material, which composition can maintain the anti -oxidization property of the color developer solution, can ensure freedom from the color density reduction and also from fogging, gives out less odor and is not a deadly compound, and also a method of process using the same composition. As a result, it was found that the above problems could be solved by permitting process with a color developer solution containing a particular composition. The present invention is predicated in this finding.

An object of the invention, accordingly, is to provide a color developer composition, which can maintain the anti-oxidization property of the color developer solution, is free from such photographic performance deterioration as the color density reduction or fogging, gives out less odor and is free from any deadly compound, and also a method of processing silver halide photographic material using the same composition.

### Description of the Invention

The constitution of the invention is the process of silver halide color photographic material and, more specifically, a color developer composition used for the process, which composition can maintain the anti-oxidization of color developer solution, is free from the photographic performance deterioration such as the color density reduction and fogging or the like, gives out less odor and is free from any deadly compound content, and a method of treading silver halide color photographic material by using the same composition

According to the present invention there is provided a colour developer composition containing a reaction product obtained by causing a reaction of an amine compound of 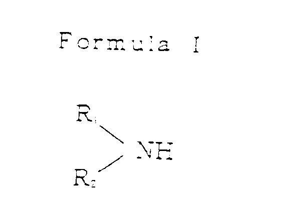
where R₁ and R₂ independently represent a member of the group consisting of a hydrogen atom, an alkyl group, an alkenyl group and an allyl group, these groups being capable of being substituted by a member of the group consisting of a hydroxyl group, an alkoxyl group and a carboxyl group, R₁ and R₂ being capable of jointly forming a hetero-ring, R₁ and R₂ being capable of forming a united bis compound, R₁ and R₂ being not both hydrogen atoms. or 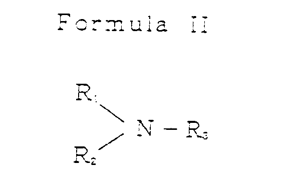
where R₁ to R₃ independently represent a member of the group consisting of an alkyl group, an alkenyl group and an allyl group, these groups being capable of being substituted by a member of the group consisting of a hydroxyl group, an alkoxyl group and a carboxyl group, R₁ and R₂ being capable of jointly forming a hetero-ring, R₁ and R₂ being capable of forming a united bis compound and hydrogen peroxide (hydrogen peroxide water) and without separation of the reaction product or by reaction of an amine compound of the Formula II and hydrogen peroxide (hydrogen peroxide water) then causing a reaction of the reacted system and an amine of the Formula I and without separation of the reaction product.

Features of the invention reside in:
(1) a color developer composition, which contains a reaction product obtained by causing a reaction between an amine compound of Formula I and hydrogen peroxide (hydrogen peroxide water), the reaction product not being seperated.
(2) a colour developer composition, which contains a reaction product obtained by causing a reaction between an amine compound of Formula II and hydrogen peroxide (hydrogen peroxide water), the reaction product not being separated.
(3) a color developer composition, which contains a reaction product obtained by causing a reaction between an amine compound of Formula II and hydrogen peroxide (hydrogen peroxide water) and then causing a reaction between the reacted system and an amine compound of Formula I, the reaction product not being separated;
(4) a method of processing a silver halide color photographic material of any constitution and composition by using a color developer solution given in (1), (2) or (3) above in a color developing process carried out after exposure of the material to a light image ; and
(5) a method of processing a silver halide color photographic material of any constitution and composition by using an automatic developing apparatus of any constitution and also using a color developer solution given in (1), (2) or (3).

While the present invention concerns reaction products, a compound which is newly obtained as a result of a reaction of a different compound, is usually separated from the reaction system. This is done so because if the resultant reaction system contains other reaction product compounds and/or compounds remaining without undergoing reaction, it is liable that the intrinsic character of the intended reaction product compound fails to be obtained, and defects of other compounds than the intended compound are produced. In view of the shelf stability, it is usually necessary to sufficiently increase the purity of the intended compound. Therefore, the resultant reaction system is subjected to separation and refinement. This means that a great amount of material is needed, and the efficiency with respect to the charged material is very inferior. The reaction product according to the invention features that all the compounds in the reaction system including reaction product compounds can be used and that these compounds are stable without separation or refinement. Thus, it is possible to obtain a very efficient reaction product.

Up to date, a large variety of silver halide photographic materials are commercially available. They are available in different forms depending on their purposes. For example, they are available as color negative films or color reversal films. As printing material, there are negative-positive and positive-positive materials. Usually, color negative films and negative-positive printing materials are used. These many different silver halide photographic materials have different laminar structures and their own features. Further, there is an extravagant variety of silver halide compositions constituting these layers. Further, the differences of the amounts of silver chloride, silver bromide and silver iodide used, differences of the composition ratio and differences of the size and shape of these silver halide grains provide for respective features. Further, various additives such as sensitivity-increasing pigments, stabilizers, sensitivity increasing agents and suppressing agents are used. The particle size is dealt with in "Basis of Photographic Industry --- Silver Salt Photography", edited by the Society of Photographic Science and Technology of Japan and published by Corona Co., Ltd., p-p. 277-278. Further, silver halide color photographic materials use various couplers. As such couplers, there is a very large variety of materials. The term "a silver halide color photographic material of any constitution and composition, used in the appended claims, refers to the above circumstances.

For processing a silver halide color photographic material, usually an automatic developing apparatus is used. There is a great variety of automatic developing apparatuses, which are different in type, speed of process, temperature of process, process steps, method of replenishment with replenishment solution, method of dealing with over-flow solution and so forth and have their own features. The term "an automatic developing apparatus of any constitution" used in the appended claims refers to the above circumstances.

The color developer composition according to the invention concerns its own performance, which is not influenced by various conditions like those noted above.

The compounds used according to the invention are exemplified below without any sense of limiting the invention. Generally, the compounds used according to the invention are represented by Formulae I and II. Examples of the compounds of the Formulae I and II are as follows:

### Compound I - 1:

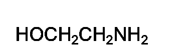

### Compound I - 2:

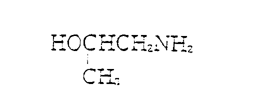

### Compound I - 3:

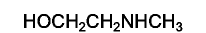

### Compound I - 4:

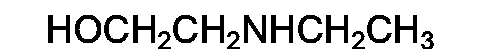

### Compound I - 5:

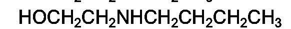

### Compound I - 6:

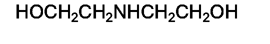

### Compound I - 7:

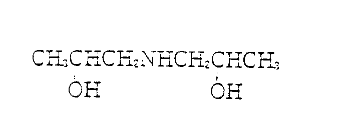

### Compound I - 8:

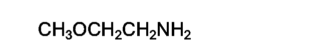

### Compound I - 9:

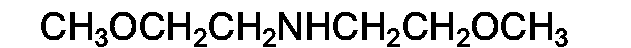

### Compound I - 10:

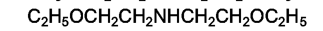

### Compound I - 11:

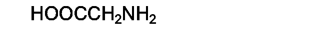

### Compound I - 12:

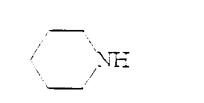

### Compound I - 13:

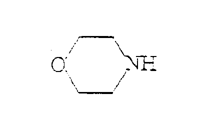

### Compound I - 14:

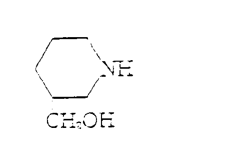

### Compound I - 15:

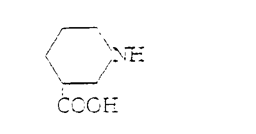

### Compound I - 16:

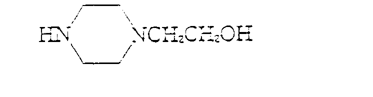

### Compound I - 17:

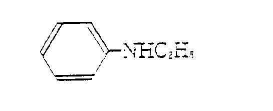

### Compound I - 18:

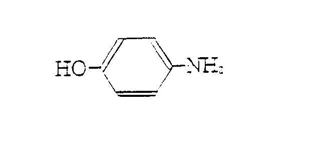

### Compound I - 19:

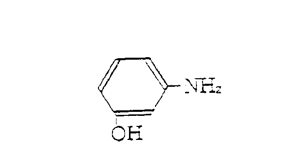

### Compound I - 20:

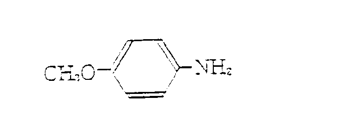

### Compound I - 21:

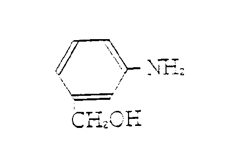

### Compound I - 22:

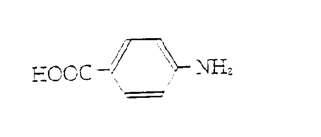

### Compound I - 23:

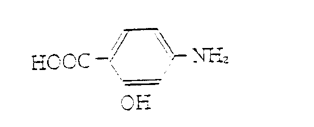

### Compound I - 24:

### Compound II - 1:

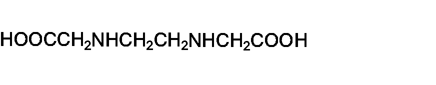

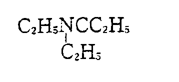

### Compound II - 2:

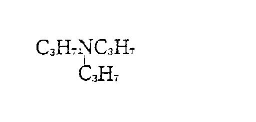

### Compound II - 3:

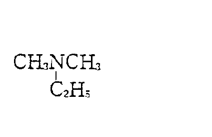

### Compound II - 4:

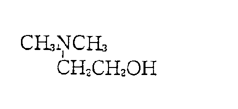

### Compound II - 5:

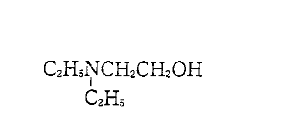

### Compound II - 6:

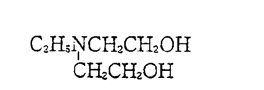

### Compound II - 7:

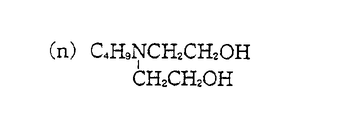

### Compound II - 8:

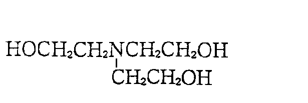

### Compound II - 9:

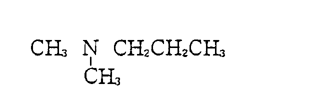

### Compound II - 10:

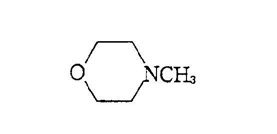

### Compound II - 11:

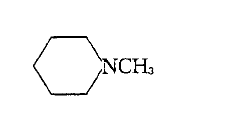

### Compound II - 12:

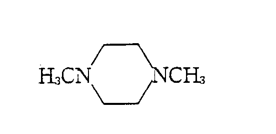

### Compound II - 13:

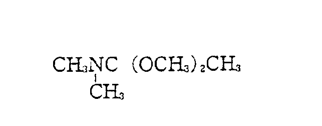

### Compound II - 14:

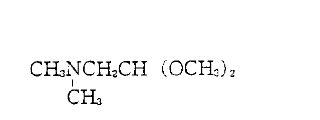

### Compound II - 15:

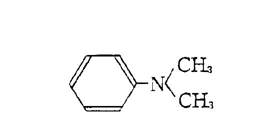

### Compound II - 16:

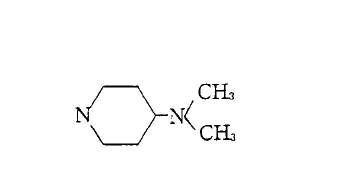

### Compound II - 17:

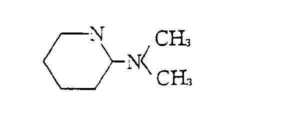

### Compound II - 18:

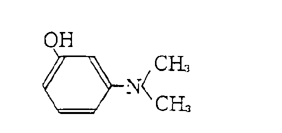

### Compound II - 19:

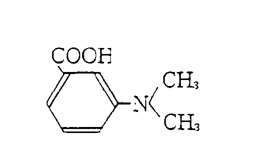

### Compound II - 20:

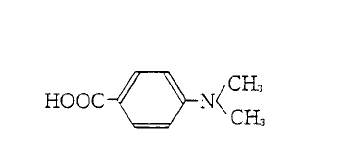

### Compound II - 21:

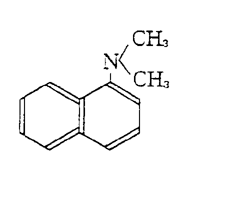

### Compound II - 22:

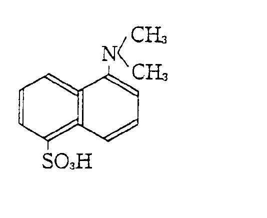

### Compound II - 23:

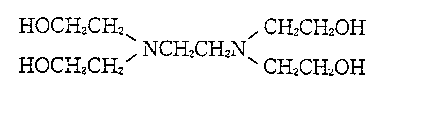

### Compound II - 24:

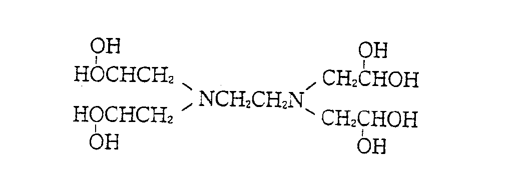

The principal color developer material used for the color developer solution according to the invention, may be any general aromatic primary amine type principal color developer material. Examples of the principal color developer material according to the invention are shown in Journal of Americal Chemical Society, 73, 3100 (1951) and Haist, "Modern Photographic Processing", 1979, John Wily and Sons, New York, p. 545 and following pages.

The color developer solution used according to the invention may contain various usual components. Examples of the components are such alkali compounds as potassium hydroxide, sodium hydroxide, potassium carbonate, sodium carbonate, potassium phosphate and sodium phosphate, such disulfates as sodium disulfate, potassium disulfate, sodium hydrosulfite, potassium hydrosulfite, sodium metabisulfite and potassium metabisulfite, such halides as hydrosulfite salts, metabisulfite salts, potassium chloride, sodium chloride, potassium bromide, sodium bromide, potassium iodide and sodium iodide, such water-softening agents as aminopolycarbonic acid, polystyrene sulfonic acid and polyphosphonic acid, such thickening agents as ethylene glycol, diethylene glycol, diethanolamine and triethanolamine and development acceleration agents. The color developer solution may further contain such additive compounds as nitrobenzoimidazol, mercaptobenzoimidazol, 5-methyl-benzotriazol and 1-phenyl-5-mercaptotetrazol and also such additives as anti-stain agents and anti-sludge agents.

The color developer solution used according to the invention suitably has a pH of 9.5 or above. Generally, a quicker process is possible by increasing the pH to a certain extent. In this case, however, the solution is denatured with the lapse of time, thus resulting in fogging or contamination of the base. With the color developer composition and method of processing according to the invention, it is possible to effect process with a composition, which permits maintaining the anti-oxidization property of the color developer solution with lapse of time, is free from color density reduction or fogging, gives out less odor and is not a deadly compound.

The bleaching material used for the bleaching solution according to the invention are metal complex salts of organic acids. Among the organic acids of the metal complex salts are aminopolycarbonic acid and citric acid, and among the metals are iron, cobalt and copper. Polycarbonic acid is the most suitable organic acid for forming such metal complex salt of organic acid. Such polycarbonic acid or aminopolycarbonic acid may be in the form of alkali metal salts, ammonium salts or water-soluble amine salts. Examples are ethylenediamine tetracetic acid, diethylenetriamine pentacetic acid, ethylenediamine ( -oxyethyl))-N,N'-triacetic acid, pro- pynediamine tetracetic acid, trinitroacetic acid, iminodiacetic acid, cyclohexanediamine tetracetic acid, hydrox- yethylglycine succinic acid, ethyletherdiamine tetracetic acid, ethylenediamine tetrapropionic acid and phenylenediamine tetracetic acid and also alkali metal salts, ammonium salts and water-soluble amine salts of these acids. Halide formers are generally halogenated hydroxylic acid and halogenated alkali metal and ammonium salts. Their examples are hydrogen chloride, hydrogen bromide, sodium chloride, potassium chloride, ammonium chloride, sodium bromide, potassium bromide and ammonium bromide. As buffer, inorganic weak acids are used in combination with organic acids and alkali compounds. Examples of the inorganic weak acid are carbonoates, borates and phosphates. Examples of the organic acid are acetic acid, citric acid and succinic acid. Specific examples of the buffer components are sodium carbonate, potassium carbonate, ammonium carbonate, sodium borate, potassium borate, sodium phosphate, potassium phosphate, ammonium phosphate, sodium acetate, potassium acetate, ammonium acetate, sodium citrate, potassium ccitrate, ammonium citrate, sodium succinate, potassium succinate and ammonium succinate. Further, bleaching promoters are used as additives. Examples of the additive are shown in Japanese Patent Application Public Disclosure No. 35727/1979, like Disclosure No. 25064/1980, like Disclosure No. 12549/1985, like Disclosure No. 76745/1985, like Disclosure No. 95540/1985, like Disclosure No. 125843/1985, like Disclosure No. 22175/1985, like Disclosure No. 230140/1985, like Disclosure No. 244950/1985, like Disclosure No. 50149/1986, like Disclosure No. 118752/1986, like Disclosure No. 80649/1987, like Disclosure No. 89963/1987, like disclosure No.1311260/1987, like Disclosure No. 135835/1987, like Disclosure No. 166344/1987, like Disclosure No. 166345/1987, like Disclosure No. 166346/1987, like Disclosure No. 166347/1987, like Disclosure No. 166348/1987, like Disclosure No. 168159/1987, like Disclosure No. 8141/1988, like Disclosure No. 73247/1988, like Disclosure No. 163853/1988, like Disclosure No. 256953/1988, like Disclosure No. 261362/1988, like Disclosure No. 20485/1989, like Disclosure No. 13550/1989, like Disclosure No. 15739/1989, like Disclosure No. 102559/1989, like Disclosure No. 170943/1989, like Disclosure No. 211757/1989, like Disclosure No. 213650/1989, like Disclosure No. 22595/1989, like Disclosure No. 245256/1989, like Disclosure No. 44349/1990, like Disclosure No. 93455/1990 and like Disclosure No. 103041/1990.

The fixing agent used for the fixing solution according to the invention is a compound which can react with silver halide to form an aqueous complex salt. It is a thiosulfate or a thiocyanate. Its examples are sodium thiosulfate, potassium thiosulfate, ammonium thiosulfate, sodium thiocyanate, potassium thiocyanate and ammonium thiocyanate. Further, it may be such compound as thiourea and thioether. The anti-oxidization agent may be sulfites, hydrosulfites, metabisulfites, etc. Its examples are sodium sulfite, potassium sulfite, ammonium sulfite, sodium hydrosulfite, potassium hydrosulfite, ammonium hydrosulfite, sodium metabisulfite and potassium metabisulfite. As the buffer is used an inorganic weak acid in combination with an organic salt and an alkali compound. Examples of the organic weak acid are carbonic acid, boric acid and phosphoric acid, and examples of the organic acid are acetic acid, citric acid and succinic acid. Specific examples of the buffer components are sodium carbonate, potassium carbonate, ammonium carbonate, sodium borate, potassium borate, ammonium borate, sodium phosphate, potassium phosphate, ammonium phosphate, sodium acetate, potassium acetate, ammonium acetate, sodium citrate, potassium citrate, ammonium citrate, sodium succinate, potassium succinate and ammonium succinate.

The bleaching agent used for the bleaching fixing solution according to the invention is also used for the bleaching solution noted above. The fixing agent is also used for the fixing solution noted above. The buffer is also used forthe bleaching solution and fixing solution noted above. Further, additives are used for promoting the bleaching and fixing. These additives are shown in Japanese Patent Publication No. 8506/1970, like Publication No. 8836/1970, Japanese Patent Application Public Disclosure No. 556/1971, Japanese Patent Publication No. 9854/1978, Japanese Patent Application Public Disclosure No. 71634/1979 and Bergien Patent No. 770910.

The water washing process according to the invention is done by water washing or a substitute stabilizing process. The substitute stabilizing process for water washing is carried out by using a stabilizing solution with a pH of 5 to 8. The stabilizing solution may contain a buffer, which may be any well-known alkali or acid compound.

Examples of the color developer composition according to the invention are obtained as follows :
(1) To 1 mol of an amine compound represented by Formula 1 is added 0.5 to 1.2 mol of hydrogen peroxide water.
(2) To 1 mol of an amine compound represented by Formula is added 1.0 to 1.2 mol of hydrogen peroxide water.
(3) To 1 mol of an amine compound represented by Formula is added 1.0 to 1.5 mol of hydrogen peroxide water, and then is added 0.5 to 1.0 mol of an amine compound represented by Formula 1.
(4) To 1 mol of hydrogen peroxide is added 0.7 to 1.0 mol of an amine compound represented by Formula II and then is added 0.3 to 1.0 mol of an amine compound represented by Formula I.

The above examples of the color developer composition are obtained in the following reaction examples :
(1) To 238 g of ethylethanolamine (1-5) is added 100 ml of water, and then 259 g of 35 % hydrogen peroxide water is added while agitating the system. The system is held at 60 °C or below and then restored to room temperature. The system is then diluted with water to 1 I.
(2) To 355 g of ethyldiethanolamine (11-6) is added 259 g of 35 % hydrogen peroxide water while agitating the system. The system is held at 60 °C or below and then restored to room temperature. The system is then diluted with water to 1 I.
(3) To 398 g of triethanolamine (11-8) is added 259 g of 35 % hydrogen peroxide water while the system is agitated at 60 °C or below. The system is then agitated at the same temperature for 30 minutes. Then, 200 g of methylethanolamine (1-3) is added, and the system is then restored to room temperature. Then, the system is diluted with water to 1 I.
(4) To 454 g of 20 % hydrogen peroxide water is added 312 g of diethylethanolamine (11-5) while the system is agitated at 60 °C. The system is then agitated at the same temperature for 30 minutes. Then, 280 g of diethanolamine (1-6) is added, and the system is then restored to room temperature. Then, the system is diluted with water to 1 I.

The reaction products obtained in the above ways can be used without any treatments as color developer compositions, which is a quite new attainment.

As shown above, the color developer solution according to the invention, in which the reaction product obtained from amine compounds and hydrogen peroxide water is used in situ as color developer composition, can maintain the anti-oxidization property, does not cause photographic performance deterioration such as the color density reduction orfogging, gives out less odor, does not contain any deadly compounds and permits a method of processing a silver halide color photographic material by using it.

Examples of the invention are given below without any sense of limiting the invention.

### Example 1

Commercially available color paper was exposed to light image and then processed under the following conditions and using the following process solutions.

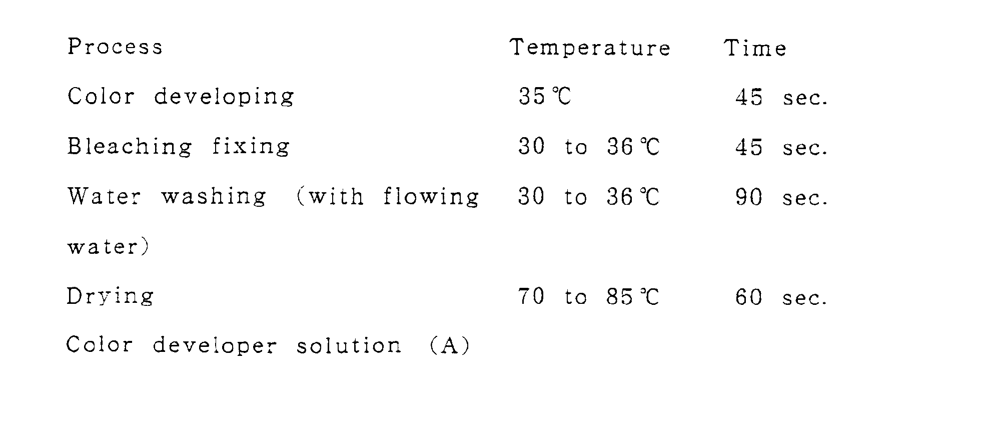

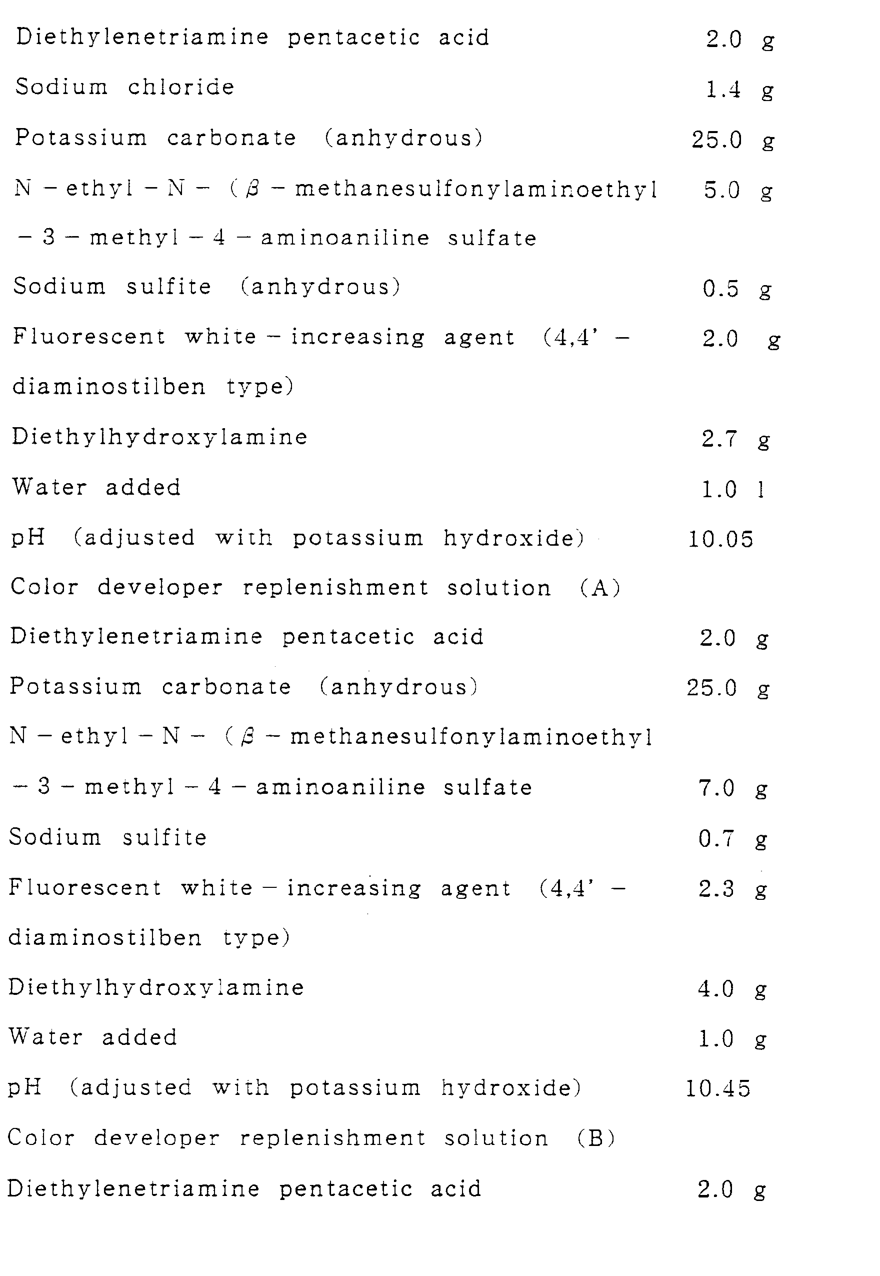

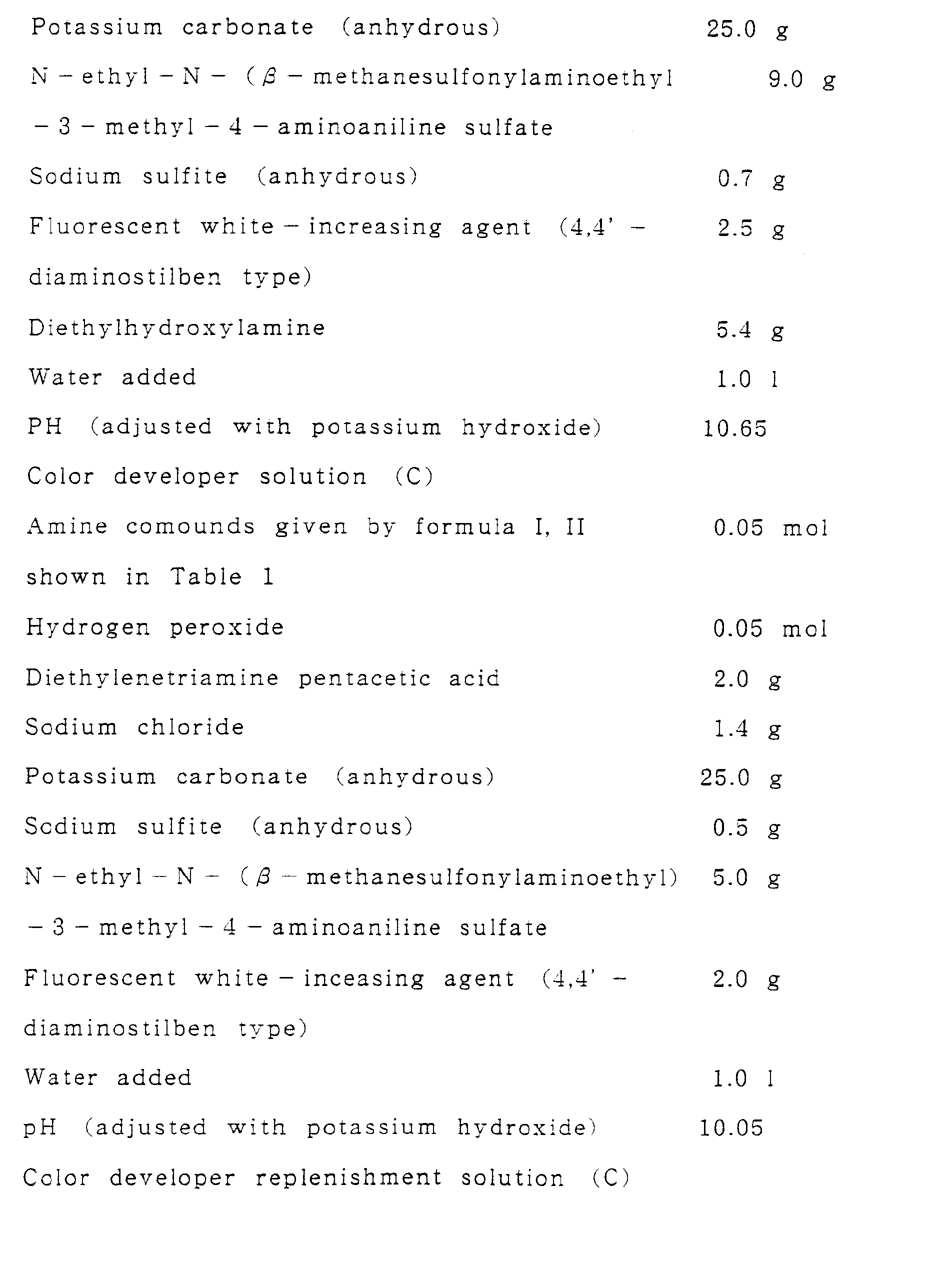

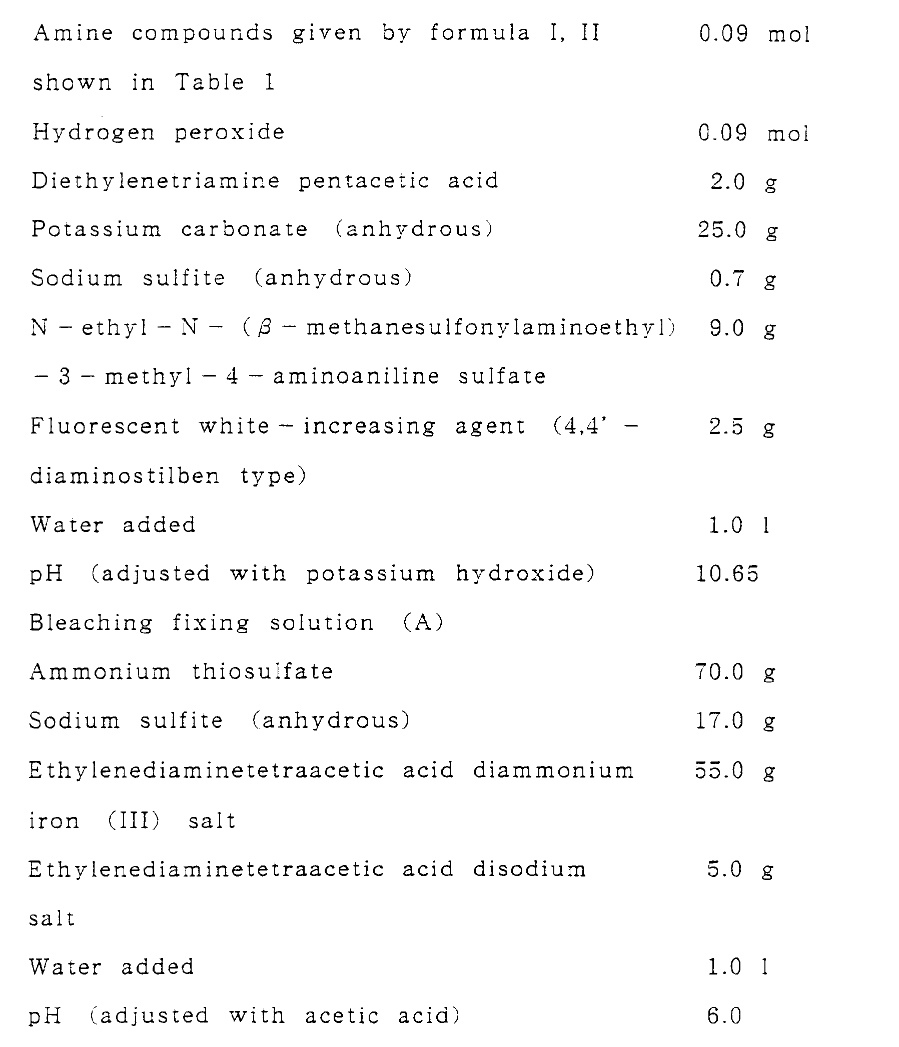

In the process, the color developer solution (A) and bleaching fixing solution (A) were charged into an automatic developing apparatus, 160 ml of the color developer replenishment solution (A) was used for 1 m² of the photographic material, 80 ml of the color developer replenishment solution (B) was used for 1 m² of the photographic material, and 220 ml of the bleaching fixing solution (A) was used for 1 m² of the photographic material. With respect to the color developer solution (C) according to the invention, 80 ml of the color developer replenishment solution (C) was used for 1 m² of the photographic material. The bleaching fixing solution was replenished in the same manner as above. The amount of the processed color paper was 15 m² per day and 150 m² in 10 days. The states at the start and end of the process were measured with a commercially available strip to determine
the minimum density charge (Δ Dₘᵢₙ), sensitivity point change (Δ LD) and contrast change (Δ HD-A LD). The measurement was made using a reflecting density meter ("X-RITE 310"). The results are as shown in Table 1.

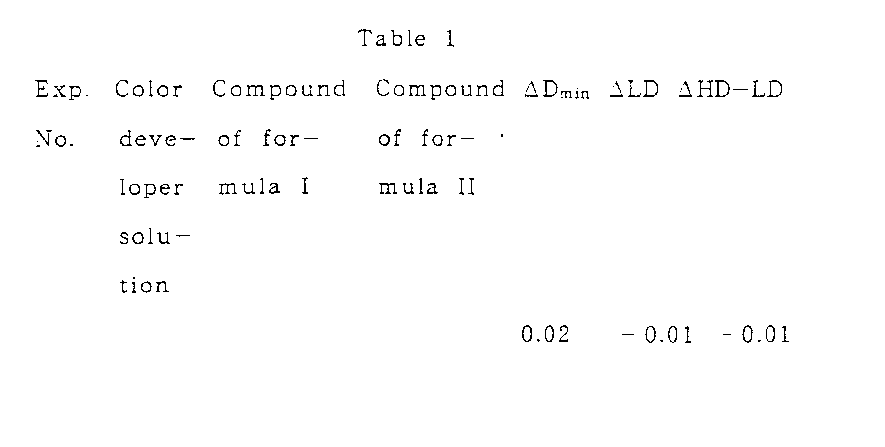

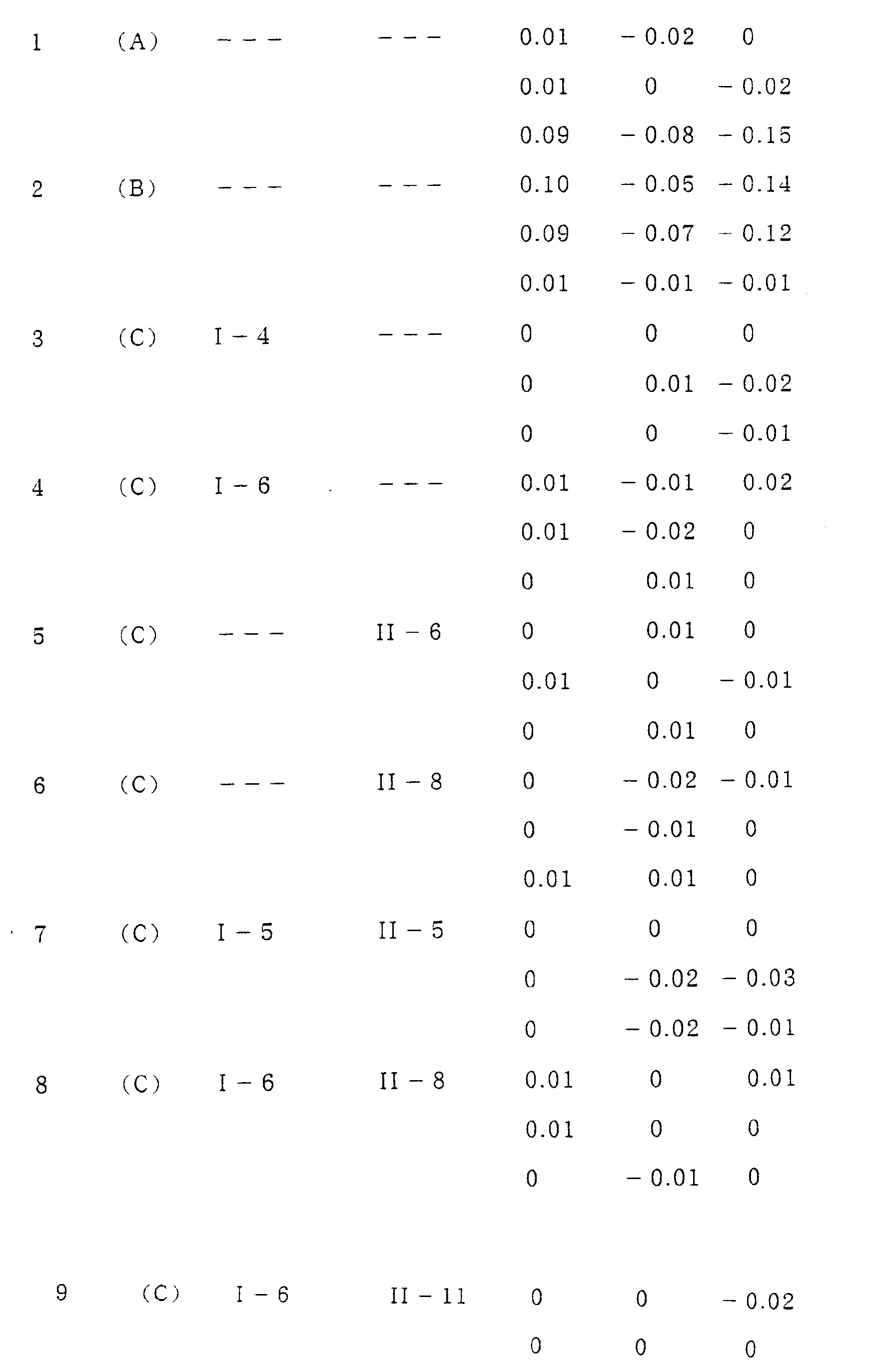

In Table 1, the figures in the three rows for each experiments show the blue, green and red color densities from the top row.

As is seen from the above results, color paper can be continuously processed without any problem if the color developer replenishment solution is used greatly (Experiment No.1). If the amount of the color developer replenishment solution used is small, however, various problems are posed such as an increase of the minimum density, a reduction of the sensitivity and a reduction of the contrast (Experiment No. 2). However, it is seen that in the method of processing using the color developer solution according to the invention, the continuous processing can be carried out stably and without any problems even if the amount of the color developer replenishment solution used is small (Experiments No. 3 to No. 9). That is, by using the color developer composition according to the invention it is possible to process a silver halide color photographic material such that the anti-oxidizaiton property of the color developer solution is maintained and that there occurs neither color density reduction nor fogging. Further, since the color developer composition gives out very less odor, it can be used in a satisfactory environmental status of use.

### Example 2

Commercially available color paper was exposed to light image and the processed under the following conditions and using the following process solutions.

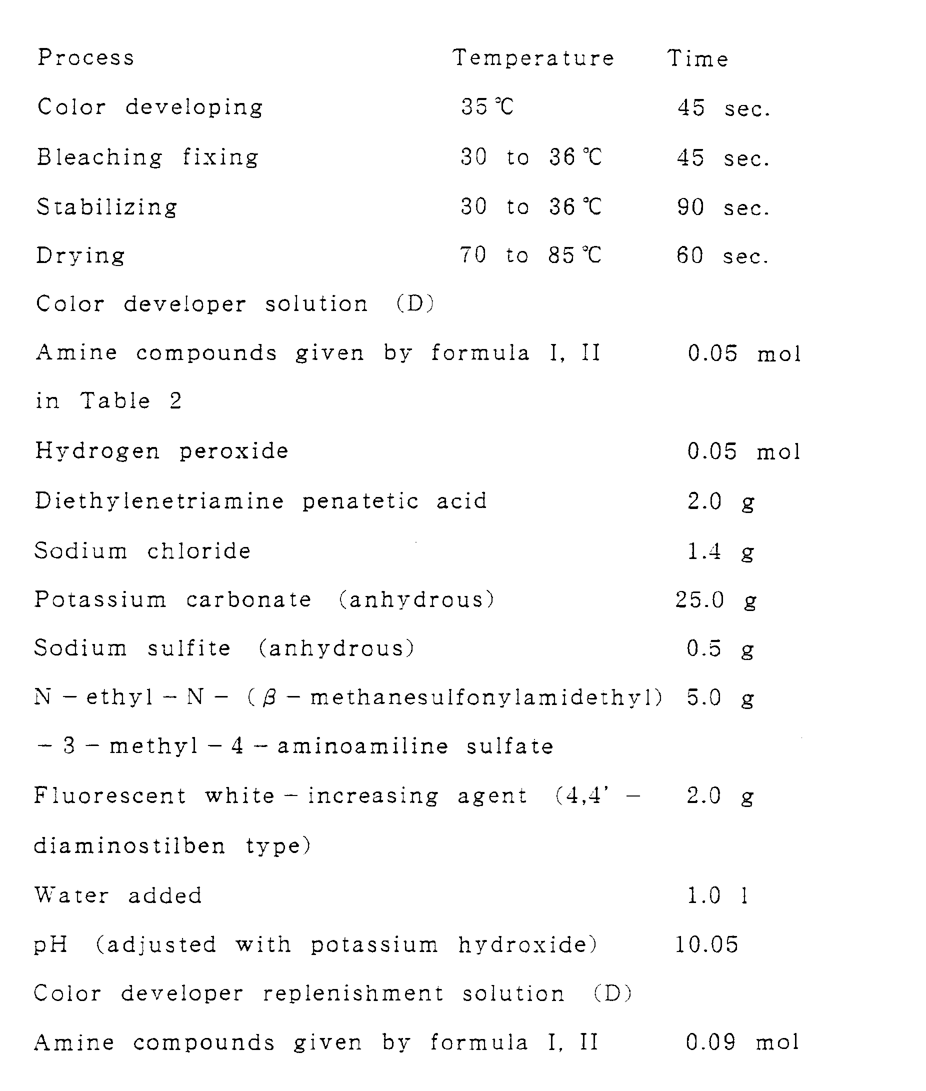

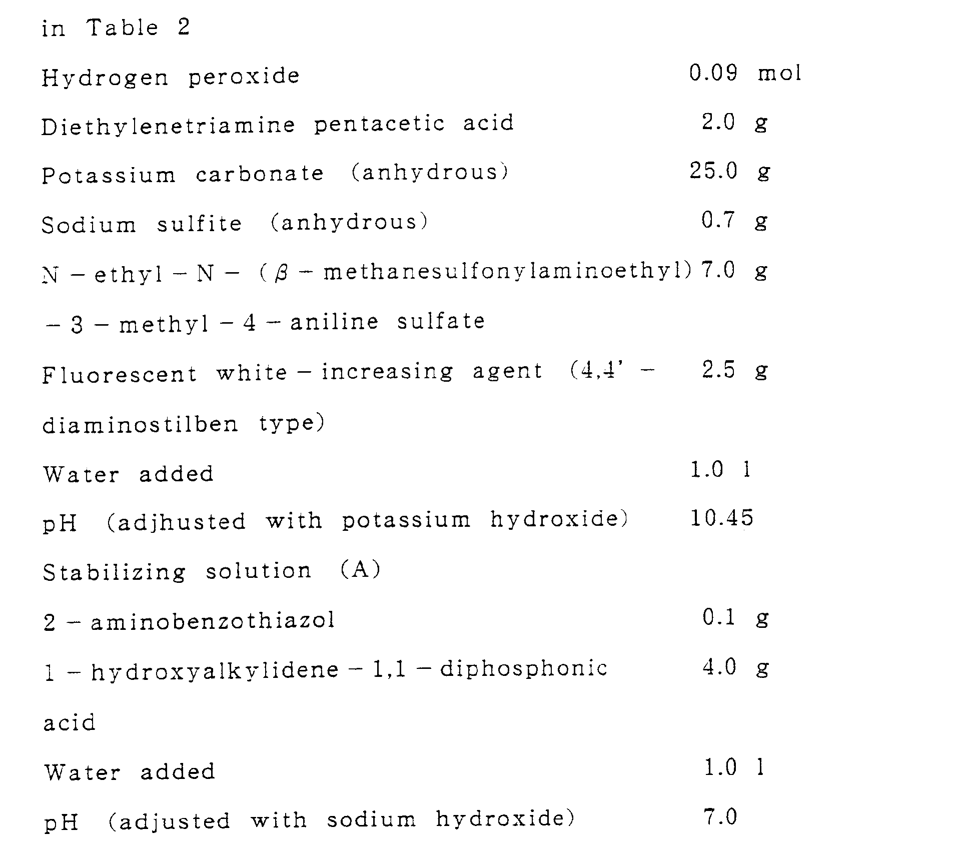

In the process, the color deveoper solution (A) in Example 1 was used, and also the bleaching fixing solution (4) and the stabilizing solution (A) were used. These solutions were charged into an automatic developing apparatus, and 160 ml of the color developer replenishment solution (A) was used for 1 m² of the photographic material. For the replenishment of the bleaching fixing solution, 220 ml of the bleaching fixing solution (A) was used for 1 m² of the photographic material, and for the replenishment of the stabilizing solution, 250 ml of the stabilizing solution (A) was used for 1 m² of the photographic material. For the replenishment of the color developer solution (D) according to the invention, 160 ml of the color developer replenishment solution (D) was used per 1 m² of the photographic material. The amount of the processed color paper were 15 m² per day and 150 m² in 10 days (process I) or 5 m² per day and 150 m² in 30 days (process II) shown in Table 2. The states at the start and end of the process were measured with a commercially available strip to determine the minimum density change (A Dₘᵢₙ), sensitivity point change (A LD) and contrast change (Δ HD-LD). The measurement was made using a reflecting density meter ("X-RITE 310"). The results are shown in Table 2.

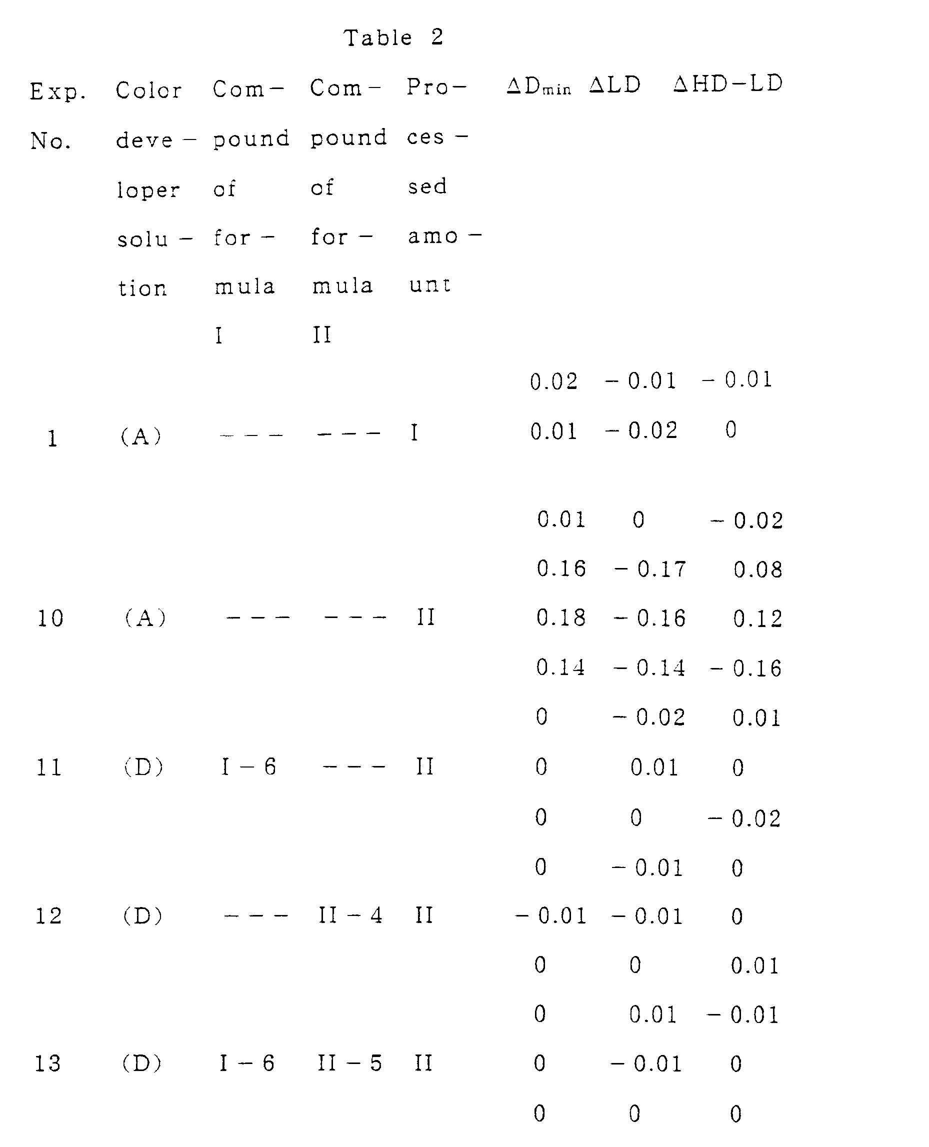

In the Table, the figures in the three rows for each experiments represent the blue, green and red color densities from the top row.

As is seen from the above results, color paper can be continuously processed without any problem if the processed amount per day is large (Experiment No.1 If the processed amount is small, however, various problems are posed such as an increase of the minimum density, a reduction of the sensitivity and a reduction of the contrast (Experiment No. 10). However, it is seen that in the method of processing using the color developer solution according to the invention, the continuous processing can be carried out stably and without any problems even if the processed amount per day is small (Experiments No. 11 to No. 13). That is, by using the color developer composition according to the invention it is possible to process a silver halide color photographic material such that the anti-oxidization property of the color developer solution is maintained and that there occurs neither color density reduction nor fogging. Further, since the color developer composition gives out very less odor, it can be used in a satisfactory environmental status of use.

### Example 3

Commercially available color negative film was exposed to light image and then processed under the following conditions and using the following process solutions.

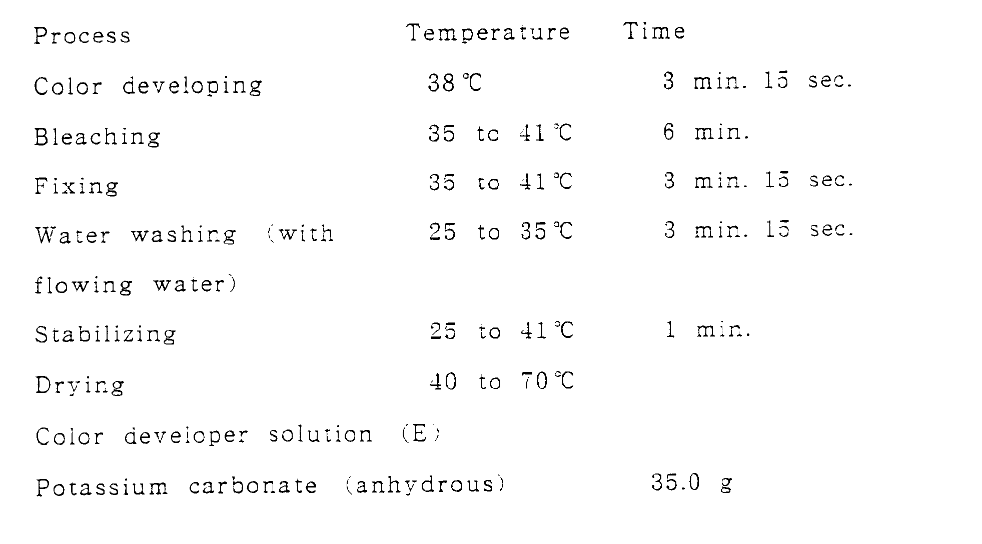

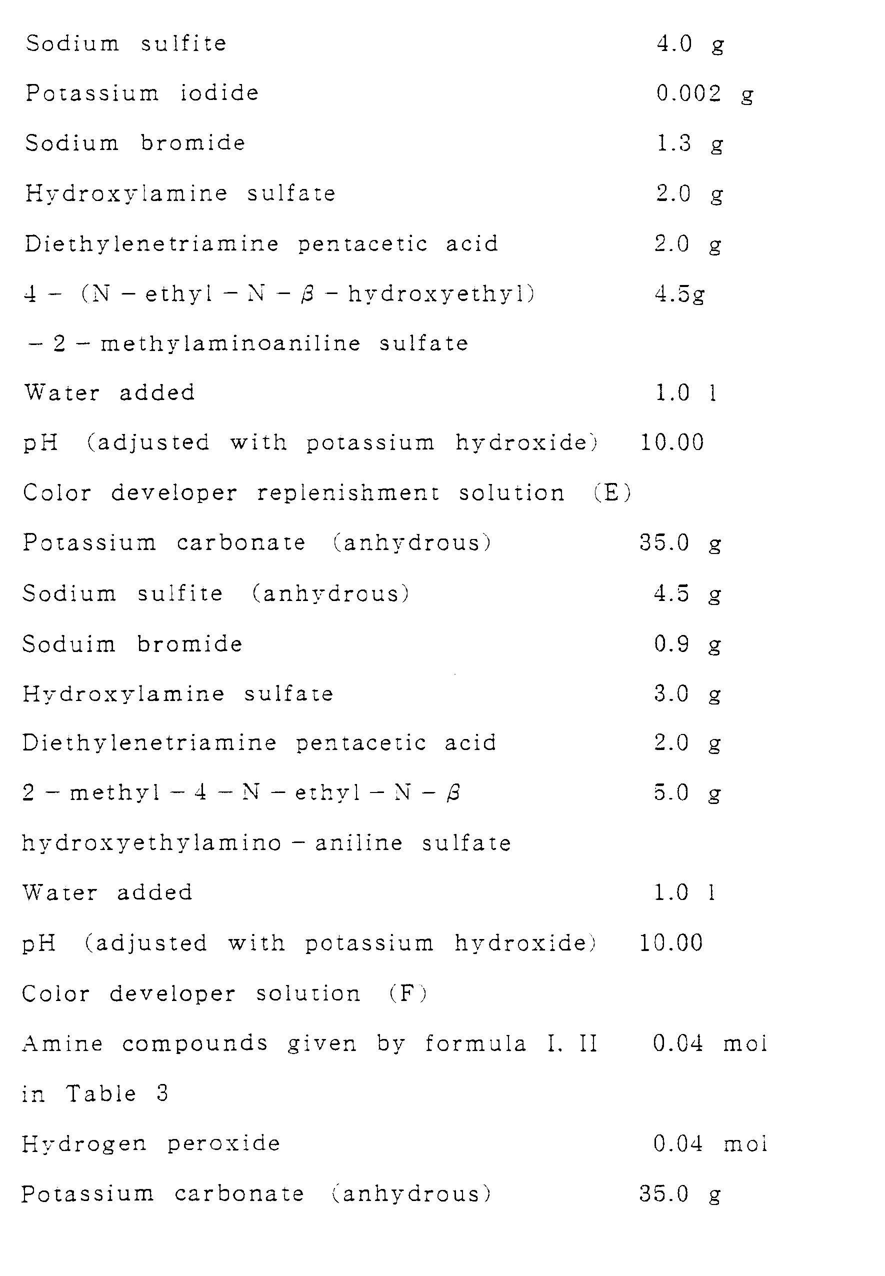

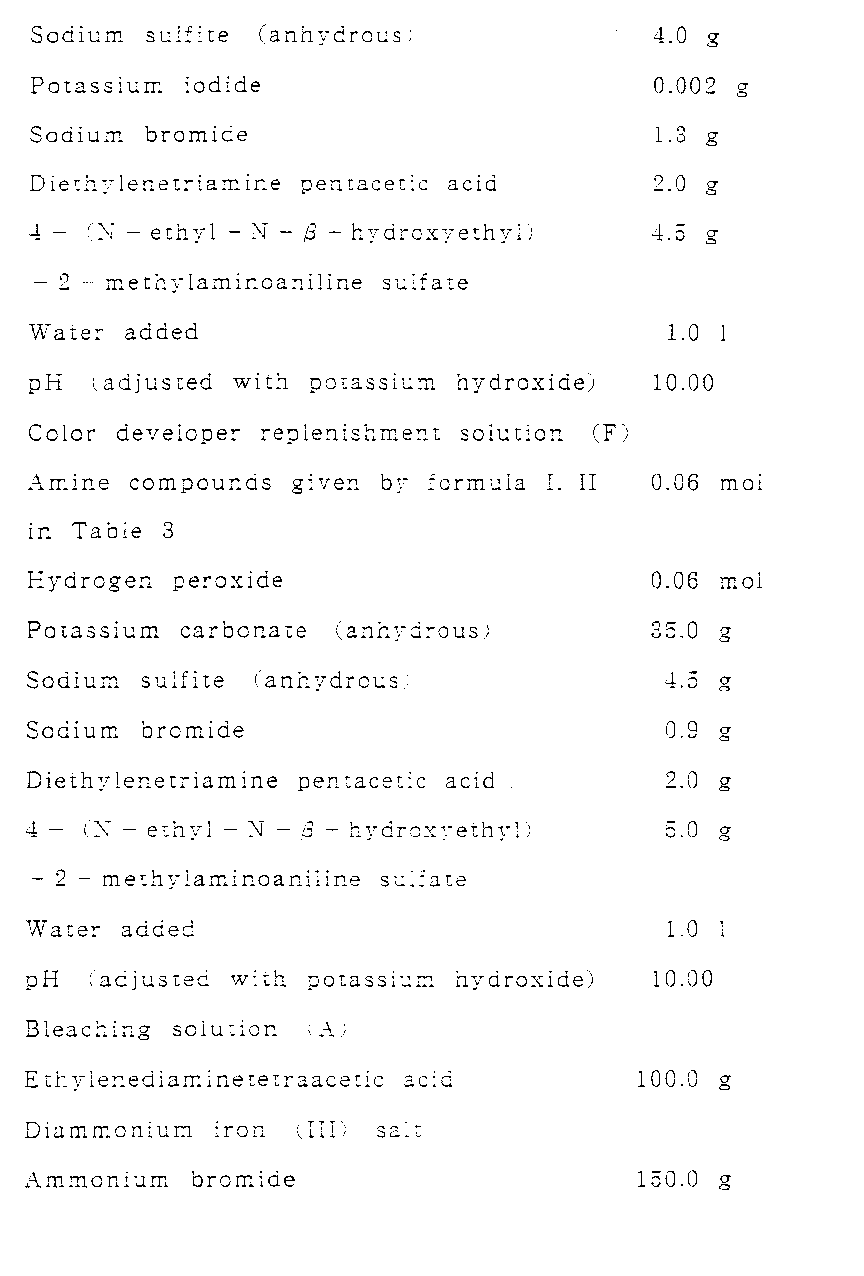

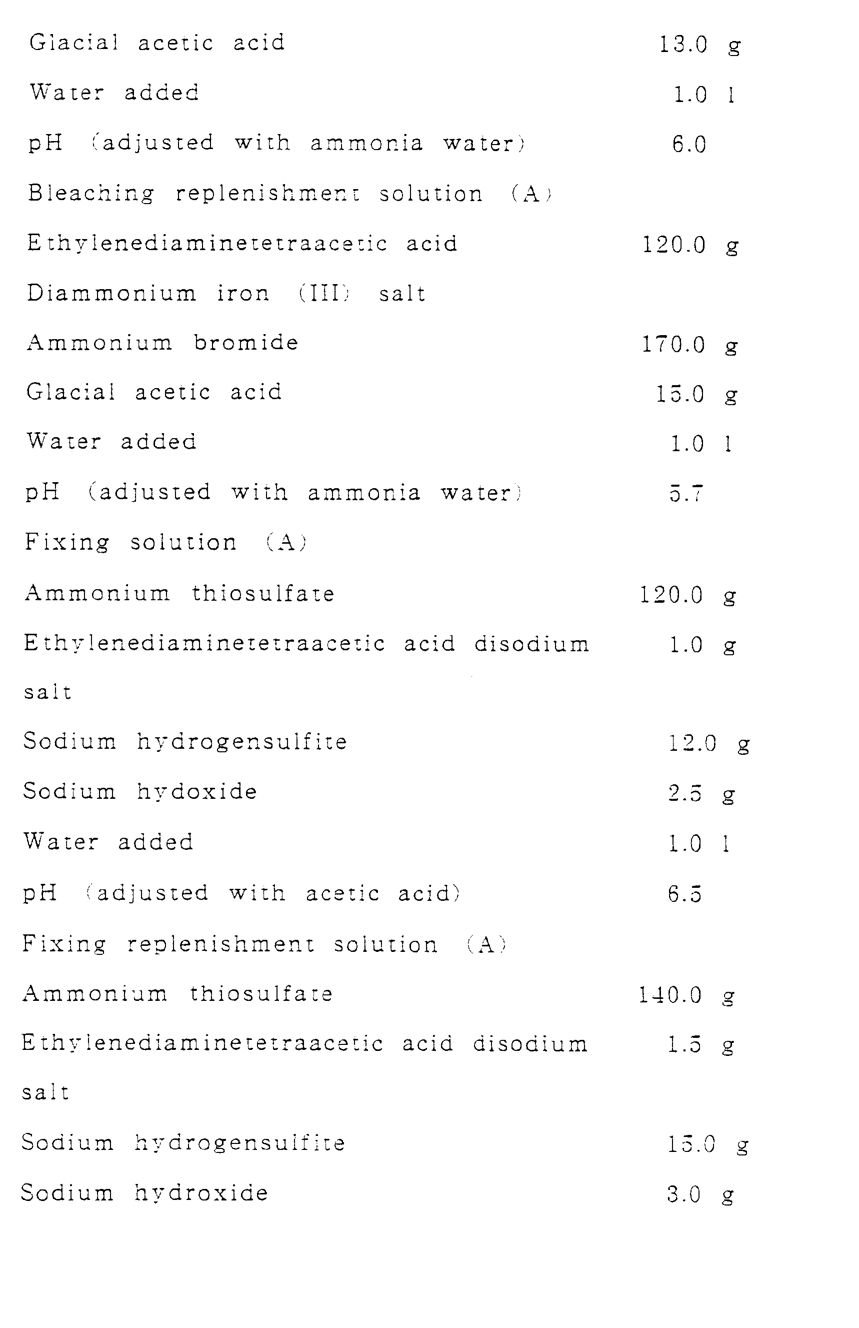

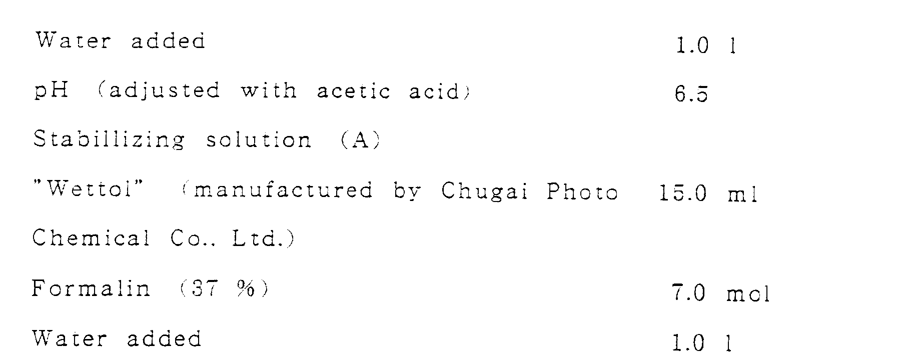

In the process, the color developer solution E), the bleaching solution (A), the fixing solution (A) and stabilizing solution (A) were charged into an automatic developing apparatus. For the replenishment of the color developer solution, 50 ml of the color developer replenishment solution (E) was used for one 135-inch 36- frames film as the photographic material. The bleaching solution was replenished with 30 ml of the bleaching replenishment solution (A) for the same amount of the photographic material. The fixing solution was replenished with 50 ml of the fixing replenishment solution (A) for the same amount of the photographic material. The stabilizing solution was replenished with 50 ml of the stabilizing solution (A) for the same amount of the photographic material. To replenish the color developer solution (F) according to the invention, 50 ml of the color developer replenishment solution (F) was used for the same amount of the photographic material. The other process solutions in this case were replenished with in the same way as above. As for the processed amount, 10 negative films were processed in a day, and 300 films in 30 days. The states at the start and end of the process were measured with a commercially available strip to determine the minimum density change (Δ Dₘᵢₙ), sensitivity point change (A LD) and contrast change (A HD-LD). The measurement was made using a reflecting density meter ("X-RITE 310"). The results are shown in Table 3.

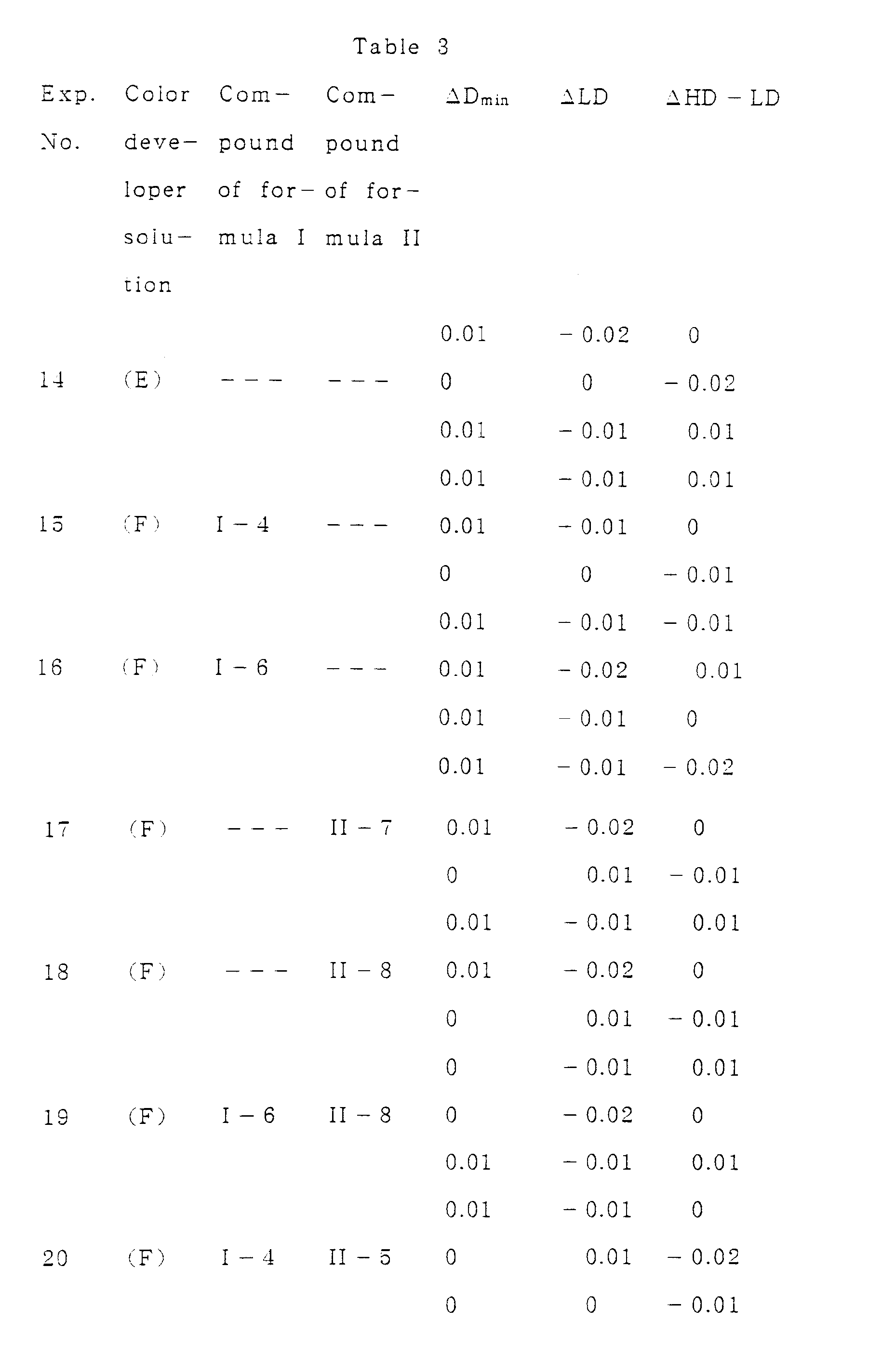

In Table 3, the figures in the three rows for each experiments show the blue, green and red color densities from the top row.

It will be seen from the above results that in the method of processing using the color developer solution according to the invention, color negative films can as well be continuously processed stably and without any problems (Experiments No. 15 to No. 20). That is, by using the color developer composition according to the invention it is possible to process a silver halide photographic material such that the anti-Oxidization property of the color developer solution is maintained and that there occurs neither color density reduction nor fogging. Further, since the color developer composition does not contain any deadly compounds. it can be used in a satisfactory environmental status of use.

### Effects of the Invention

As has been shown in the foregoing, according to the invention continuous and stable color developing process is possible on a silver halide color photographic material by using a reaction product for color developer solution, which contains all the reaction product compounds prevailing after a reaction caused between amine compounds given by formula I, II as in the Separate Sheets and hydrogen peroxide, such that the anti-oxidizing property of the color developer solution can be maintained and that neither color density reduction nor fogging occurs. Besides, the color developer composition gives out less odor and does not contain any deadly compounds, thus can be used in a satisfactory environmental status of use.

## Claims

1. A color developer composition containing a reaction product obtained by causing a reaction of an amine compound of 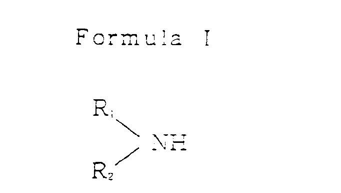
where R₁ and R₂ independently represent a member of the group consisting of a hydrogen atom, an alkyl group, an alkenyl group and an allyl group, these groups being capable of being substituted by a member of the group consisting of a hydroxyl group, an alkoxyl group and a carboxyl group, R₁ and R₂ being capable of jointly forming a hetero-ring, R₁ and R₂ being capable of forming a united bis compound, R₁ and R₂ being not both hydrogen atoms. or 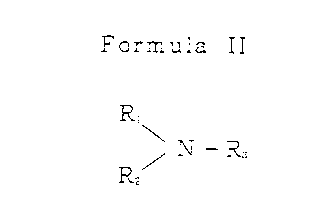
where R₁ to R₃ independently represent a member of the group consisting of an alkyl group, an alkenyl group and an allyl group. these groups being capable of being substituted by a member of the group consisting of a hydroxyl group, an alkoxyl group and a carboxyl group, R₁ and R₂ being capable of jointly forming a hetero-ring, R₁ and R₂ being capable of forming a united bis compound and hydrogen peroxide (hydrogen peroxide water) and without separation of the reaction product or by reaction of an amine compound of the Formula II and hydrogen peroxide (hydrogen peroxide water) then causing a reaction of the reacted system and an amine of the Formula I and without separation of the reaction product.

2. A composition as claimed in claim 1 wherein the amine compound of Formula 2 is selected from compounds of the following formulae:
Compound I - 1: HOCH₂CH₂NH₂
Compound I - 2: 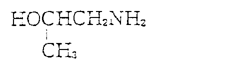
Compound I - 3: 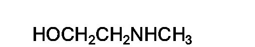
Compound I - 4:
HOCH₂CH₂NHCH₂CH₃
Compound I - 5:
HOCH₂CH₂NHCH₂CH₂CH₂CH₃
Compound I - 6: 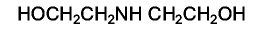
Compound I - 7: 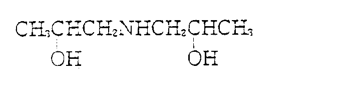
Compound I - 8: 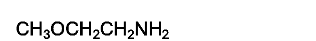
Compound I - 9: 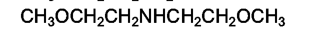
Compound I - 10: 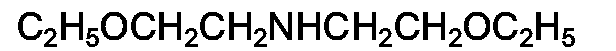
Compound I - 11: 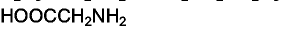
Compound I - 12: 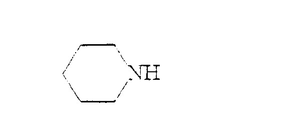
Compound I - 13: 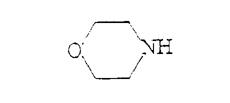
Compound I - 14: 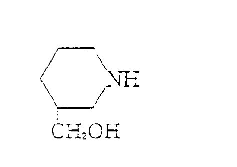
Compound I - 15: 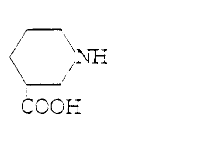
Compound I - 16: 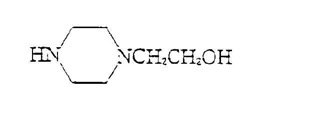
Compound I - 17: 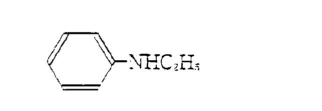
Compound I - 18: 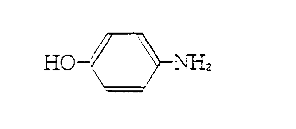
Compound I - 19: 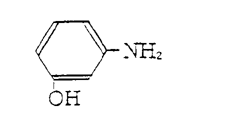
Compound I - 20: 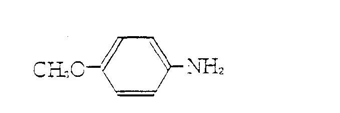
Compound I - 21: 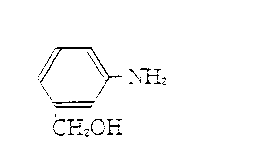
Compound I - 22: 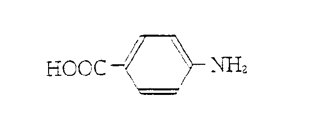
Compound I - 23: 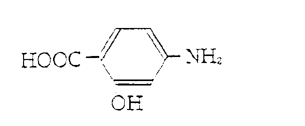
Compound I - 24:

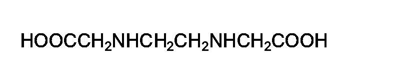

3. A composition as claimed in claim 1 or 2, wherein the amine compound of Formula II is selected from compounds of the following Formulae:
Compound II - 1: 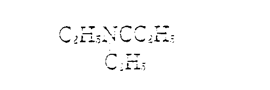
Compound II - 2: 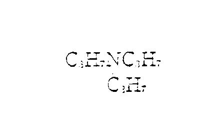
Compound II - 3: 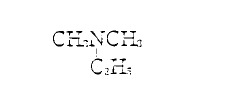
Compound II - 4: 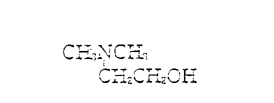
Compound II - 5: 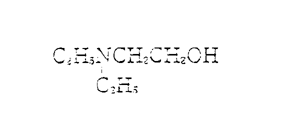
Compound II - 6: 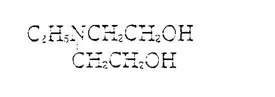
Compound II - 7: 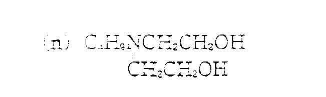
Compound II - 8: 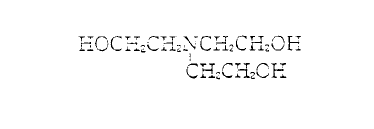
Compound II - 9: 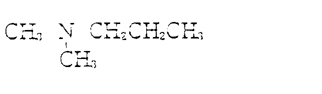
Compound II - 10: 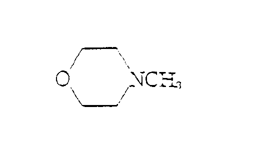
Compound II - 11: 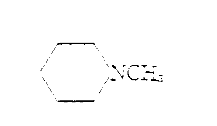 Compound II - 12: 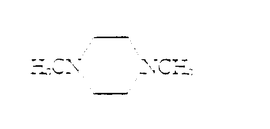
Compound II - 13: 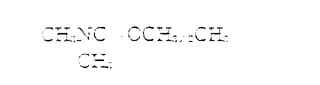
Compound II - 14: 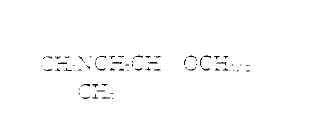
Compound II - 15: 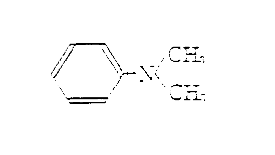
Compound II - 16: 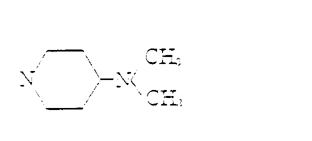
Compound II - 17: 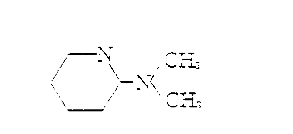
Compound II - 18: 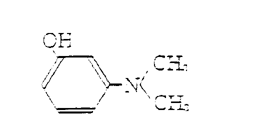
Compound II - 19: 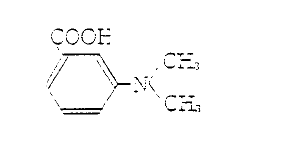
Compound II - 20: 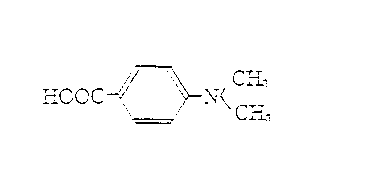
Compound II - 21: 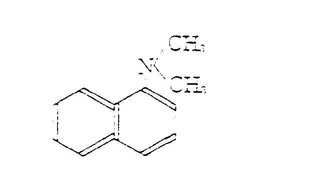
Compound II - 22: 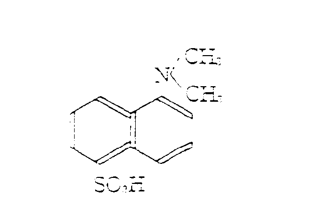
Compound II - 23: 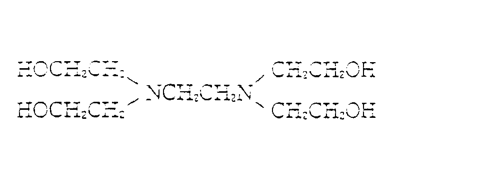
Compound II - 24:

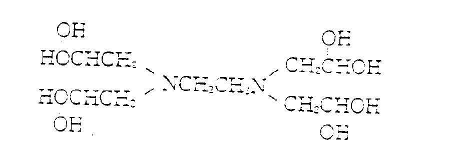

4. A method of processing a silver halide color photographic material of any constitution and composition by using a color developer solution containing a reaction product according to claim 1,2 or 3 in a color developing process carried out after exposure of said material to a light image.

5. A method as claimed in claim 4 carried out in an automatic developing apparatus.
